# EUROPEAN PATENT SPECIFICATION

(11) **EP 2 386 275 B1**
(45) Date of publication and mention of the grant of the patent: **10.10.2018**
(21) Application number: 11166127.8
(22) Date of filing: 13.05.2011
(51) Int. Cl.: A61F 2/90, A61F 5/00

(54) **Modular stent for implantation into the stomach**
Modularer Stent zur Implantation in den Magen
Extenseur modulaire apte pour l'implantation dans l'estomac

(30) Priority: 14.05.2010 KR 20100045340
(43) Date of publication of application: 16.11.2011
(73) Proprietor: Taewoong Medical Co., Ltd., Kyunggi-do 415-873 (KR); Shin, Kyong-Min, Seoul (KR)
(72) Inventor: Shin, Kyong-Min, Seoul (KR); Myung, Byung-Cheol, Kyongki-do (KR)
(74) Representative: Haseltine Lake LLP

(56) References cited:
- EP-A1- 2 085 050
- WO-A1-2009/130619
- WO-A2-2006/124541
- US-A1- 2004 172 142

## Description

### BACKGROUND OF THE INVENTION

### 1. Field of the Invention

The present invention relates generally to a stent which is implanted in the stomach of a patient who has undergone a gastrectomy so that when the patient takes in food, the stent prevents an inflammation from occurring on the suture region of the stomach that would result in contact being made with the food, and prevents the suture region from being reopened by an inflammation or a ligation of the suture region being damaged, thus reducing the time required for the suture region to heal and, more particularly, to a stent which is configured such that food that has passed through the stomach is prevented from mixing with bile or pancreatic juice in the duodenum and is directly moved into the small intestine to prevent the duodenum from absorbing the nutrients of the food while the small intestine directly digests the food and absorbs the nutrients, thus minimizing a nutrient absorption rate, thereby preventing the obesity of the patient.

### 2. Description of the Related Art

Generally, BMI (body mass index, kg/m²) is defined as the individual's body weight divided by the square of his or her height. In the case of Orientals, when the BMI is 30 or more, it is called extremely obese.

Furthermore, not only weight-related factors but also other factors including the percentage of visceral fat, an abdominal fat percentage and a body fat percentage are measured to eventually result in a diagnosis of extreme obesity.

Such an extremely obese patient may have many health problems. For example, the representative complications of the incidence of diabetes, hyperpiesia, hyperlipidemia, a fatty liver, etc. can rapidly increase in the extremely obese.

Of course, extreme obesity also rapidly increases the mortality rate resulting from such diseases as well as the incidence thereof, compared to simple obesity. In terms of reducing the weight, achieving the aim of weight-loss for the extremely obese is rapidly reduced in probability, in terms of controlling the size of meals with exercise and staying in the weight-lost state for a long period of time, compared to that of simple obesity.

Statistically, extremely obese patients ordinarily try extreme weight-loss programs, but these easily cause the problem of there being a relapse into an extremely obese state.

Such extremely obese patients may be treated by a surgical method. The following cases are among those which are candidates for the surgical treatment: a patient who has experienced failure with typical weight-loss methods, such as alimentotherapy, exercise, and correction of behavior; a patient who has a BMI of 30 or more accompanied by diabetes, hypertension, a high cholesterol disorder, a fatty liver, joint inflammation, a sleep apnea disorder, etc.; a patient who is ultra-extremely obese with a BMI of 35 or more; and a patient who has morbid abdominal obesity which is one of the characteristic obesity styles of Orientals.

Gastrectomy is a representative example of the surgical treatment method. The gastrectomy is a surgical method of turning the stomach into a tube shape in such a way as to reduce a greater curvature portion of the stomach along a lesser curvature portion, while retaining the vestibular region (antrum: the lowermost portion) which governs important digestive functions.

A patient who has undergone such a gastrectomy can easily experience satiety despite eating a small amount of food. Hence, the amount of meals is reduced so that voracious eating can be prevented. The patient who had a morbid and compulsive appetite before the gastrectomy can have normal appetite.

However, after the gastrectomy, the patient must be hospitalized or receive outpatient treatment, and there is the probability of emesis, diarrhea, laparocele, an infection, pneumonia, a respiratory disease, etc. In addition, if inosculation between the stomach and the intestine is defective, digestive fluid may leak out of the stomach.

Furthermore, such problems increase medical expenses because additional expenses resulting from complications may exceed the expenses of the operation and follow-up treatment expenses.

Moreover, when the patient who has received a gastrectomy takes in food, the food comes in contact with the suture region, thus causing pain. If the suture region is contaminated by food, the suture region may become inflamed. If the suture region is reopened by the inflammation or by damage done to the ligation region after the surgery, the patient has to get surgery again.

Meanwhile, in addition to preventing extreme obesity with a gastrectomy, a stent may be used in such a way that food which has passed through the stomach is prevented from mixing with bile or pancreatic juice so that the duodenum cannot absorb the nutrients of the food.

EP 2085050 discloses a biodegradable double stent used for various organs such as a biliary tract, an esophagus, an airway and a ureter. EP 2085050 discloses a biodegradable stent having a hollow cylindrical body woven out of a separate wire made of a biodegradable polymer so as to have a plurality of rhombic spaces fixed to an intermediate portion of a primary stent having a cylindrical body. When administered into the organ, the biodegradable stent has its original function of expanding the organ, and is firmly supported in the inner wall of a narrowed passage of the organ by pressurizing the inner wall of the narrowed passage of the organ. After a predetermined time period has elapsed, the biodegradable stent is gradually degraded away by body fluids, thereby enabling the primary stent to be easily removed from the organ.

### SUMMARY OF THE INVENTION

Accordingly, the present invention has been made keeping in mind the above problems occurring in the prior art, and an object of the present invention is to provide a stent which is configured such that the stomach of a patient who has undergone a gastrectomy is not brought into direct contact with food, thus preventing the suture region from being contaminated, thereby preventing the suture region from becoming inflamed, and preventing the suture region from being reopened by the inflammation or damage done to the ligation region after the surgery.

Another object of the present invention is to provide a stent which prevents food from coming contact with the suture region so as to avoid the occurrence of an inflammation, thus reducing the time of treatment.

A further object of the present invention is to provide a stent which includes a medical film which is made of PTFE or silicone, thus preventing food from leaking out of the stent, and enhancing the support performance of the stent.

Yet another object of the present invention is to provide a stent which may be implanted before conducting the gastrectomy and is configured such that food that has passed through the stomach is prevented from mixing with bile or pancreatic juice in the duodenum and is directly moved into the small intestine to prevent the duodenum from absorbing the nutrients of the food while the small intestine directly digests the food and absorbs the nutrients, thus minimizing a nutrient absorption rate, thereby preventing the obesity of the patient.

In order to accomplish the above object, the present invention provides a stent according to claim 1.

### BRIEF DESCRIPTION OF THE DRAWINGS

The above and other objects, features and advantages of the present invention will be more clearly understood from the following detailed description taken in conjunction with the accompanying drawings, in which:
FIG. 1 is an exploded perspective view of a stent according to the present invention;
FIG. 2 is a perspective view of the stent according to the present invention;
FIG. 3 is a front view of the stent according to the present invention;
FIG. 4 is a perspective view of the stent provided with a plurality of stent supports according to the present invention;
FIG. 5A is a partial sectional view showing a shape-memory alloy stent wherein a first film is formed inside a first wire made of PTFE according to the present invention;
FIG. 5B is a partial sectional view showing a shape-memory alloy stent wherein the first film is formed outside the first wire made of PTFE according to the present invention;
FIG. 6 is a partial sectional view showing a second or third medical silicone film formed on a wire forming an expanded diameter part or a support stent while closing openings according to the present invention; and
FIG. 7 is a view showing the stent of the present invention implanted in the stomach and the duodenum of a patient after or before gastrectomy is conducted.

### DESCRIPTION OF THE PREFERRED EMBODIMENTS

Hereinafter, a preferred embodiment of the present invention will be described in detail with reference to the attached drawings.

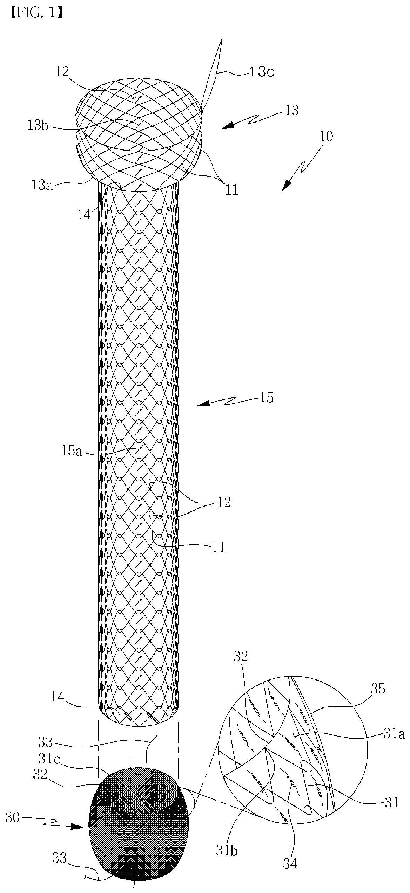

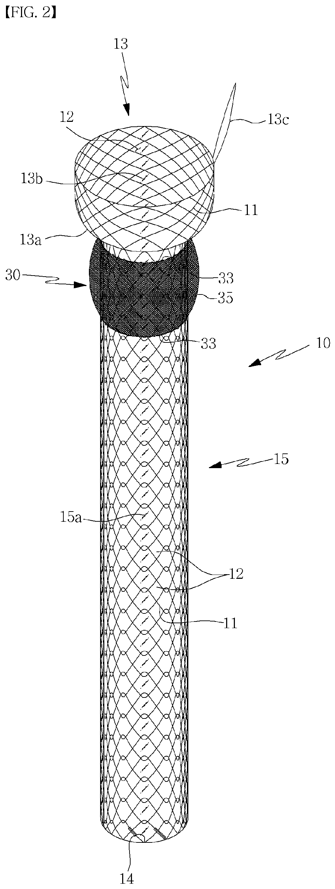

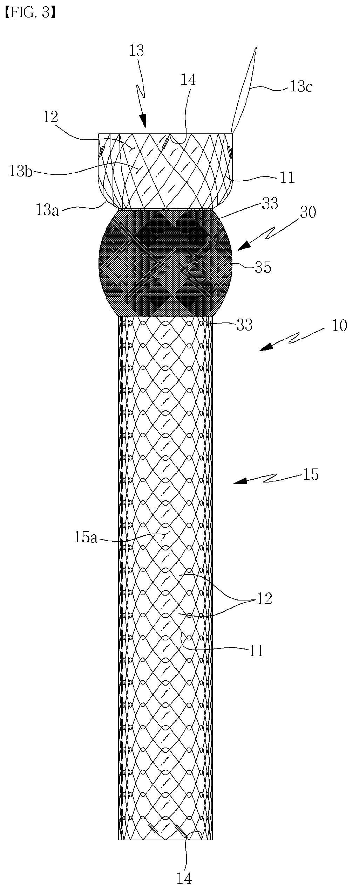

As shown in FIGS. 1 through 3, a stent according to the present invention comprises a shape-memory alloy stent 10 which is formed by weaving a piece of or a plurality of wires 11 made of material such as a super-elastic shape memory alloy such that a plurality of diamond-shaped openings 12 are formed in the stent 10. The shape-memory alloy stent 10 has a hollow cylindrical stent body 15 and an expanded diameter part 13 which is formed on an end of the cylindrical stent body 15. Bent portions 14 are formed along the circumferences of both ends of the cylindrical stent body 15.

The surface of the cylindrical stent body 15 is coated with a first medical film 15a.

Particularly, when the first medical film 15a is formed on the circumferential inner surface of the cylindrical stent body 15, flow friction is reduced which is generated, for example, when contact occurs between the wires 11 and food which has passed through the esophagus 200. When the first medical film 15a is formed on the circumferential outer surface of the cylindrical stent body 15, the wire 11 is prevented from coming into direct contact with the inner surface of a stomach 300.

Furthermore, the expanded diameter part 13 has a rounded portion 13a which is provided on a junction between the expanded diameter part 13 and the cylindrical stent body 15.

The surface of the expanded diameter part 13 is coated with a second medical film 13b.

A removal string ring 13c is connected to the bent portions 14 of the expanded diameter part 13.

The stent of the present invention further includes a support stent 30 which has an elastic spherical structure and is formed by weaving a piece of or a plurality of support stent wires 31 made of material such as a super-elastic shape memory alloy or by threading the support stent wires 31 to each other in a zigzag manner such that a hollow hole 32 is formed through the central portion of the elastic spherical structure.

The support stent 30 is fitted, through the hollow hole 32, over the circumferential outer surface of the cylindrical stent body 15 behind the expanded diameter part 13. Both ends of the support stent 30 are connected to the cylindrical stent body 15 by coupling wires 33. The surface of the support stent 30 is coated with a third medical film 34.

In detail, the support stent 30 comprises a support stent body 31c which is formed by weaving the support stent wire 31 or by threading support stent wire 31 in a zigzag manner such that openings 31a are formed in the support stent body 31c and that bent portions 31b are formed along the circumferences of both ends of the support stent body 31c.

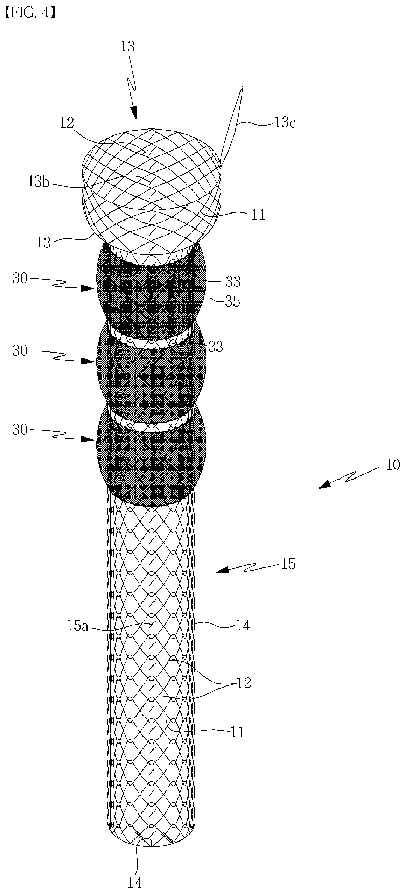

As shown as FIG. 4, a mesh of the support stent 30 formed by weaving the support stent wire 31 is smaller than that of the cylindrical stent body 15 formed by weaving the wires 11, so that the support stent 30 has comparatively superior elasticity. The stent of the present invention may include a plurality of support stents 30.

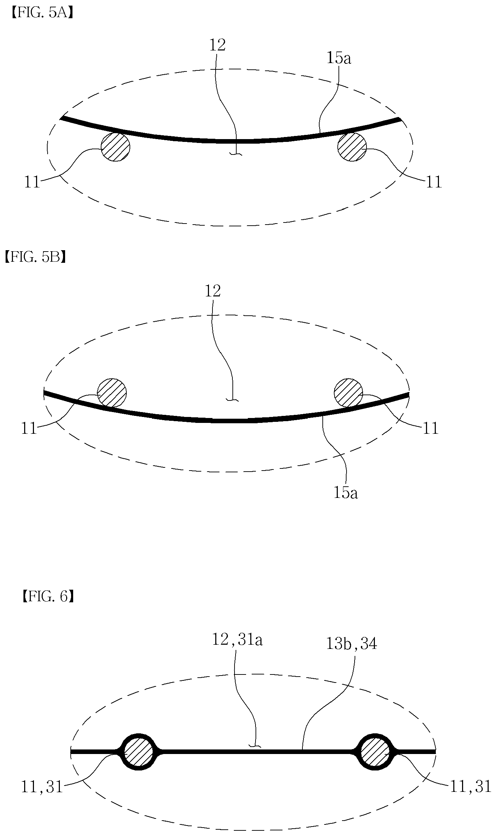

As shown in FIGS. 5A and 5B, the first medical film 15a of the shape-memory alloy stent 10 may be provided on the circumferential inner surface of the wires 11 (refer to FIG. 5A) or, alternatively, it may be provided on the circumferential outer surface of the wires 11 (refer to FIG. 5B) .

As shown in FIG. 6, the second and third medical films 13b and 34 are respectively formed on the expanded diameter part 13 and the support stent 30 by immersing the expanded diameter part 13 and the support stent 30 in liquefied silicone and hardening them such that the films cover the surfaces of the wires 11 and the support stent wires 31 and the openings defined by the wires 11 and the support stent wires 31.

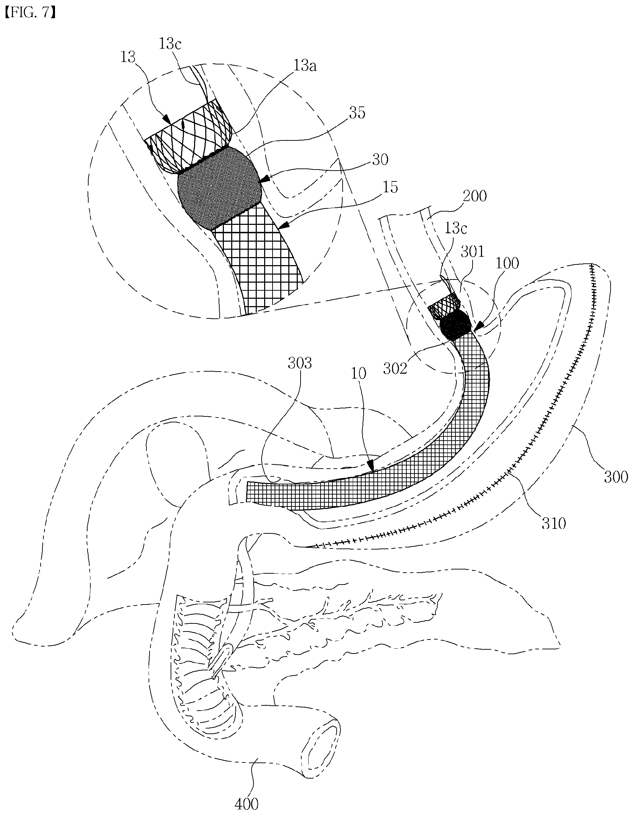

As shown in FIG. 7, the rounded portion 13a of the expanded diameter part 13 is caught and supported by the inner surface of a connection duct 301 which connects the esophagus 200 to the stomach 300.

In addition, a spherical convex surface 35 of the support stent 30 is supported by a curved inner surface of a cardiac orifice 302 of the stomach 300.

The shape-memory alloy stent 10 is inserted into a pylorus 303 via the cavity of the stomach 300.

The operation of the shape-memory alloy stent 10 of the present invention having the above-mentioned construction will be described.

In an operation of implanting the shape-memory alloy stent 10, a separate stent operation apparatus, such as a catheter, (not shown) is used.

Referring to FIGS. 1 and 7, first, the shape-memory alloy stent 10 is inserted into the catheter with a contracted volume and thereafter is inserted into the esophagus 200 by the catheter.

Then the extended diameter part 13 of the shape-memory alloy stent 10 is caught and supported by the inner surface of the connection duct 301 connecting the esophagus 200 with the stomach 300.

In addition, the spherical convex surface 35 of the support stent 30 is supported by the curved inner surface of the cardiac orifice 302 of the stomach 300.

The lower end of the shape-memory alloy stent 10 that is opposite to the expanded diameter part 13 is inserted into the pylorus 303 via the cavity of the stomach 300.

In this embodiment, the rounded portion 13a of the expanded diameter part 13 is rounded in such a way that the diameter thereof is reduced to the end connected to the shape-memory alloy stent 10 so that the rounded portion 13a is smoothly held and supported by the inner surface of the connection duct 301 that connects the esophagus 200 to the stomach 300.

Thereby, the rounded portion 13a of the expanded diameter part 13 is brought into close contact with the inner surface of the connection duct 301 without any gap being left between them. Therefore, food which has passed through the esophagus 200 is prevented from entering between the expanded diameter part 13 and the inner surface of the cardiac orifice 302.

Moreover, the support stent 30 is also brought into close contact with the inner surface of the cardiac orifice 302 so that the support stent 30 reliably supports the expanded diameter part 13, thus completing the implanting of the shape-memory alloy stent 10.

After the implanting of the shape-memory alloy stent 10 has been completed, food which is supplied into the esophagus 200 moves into the expanded diameter part 13.

Here because the shape-memory alloy stent 10 is configured such that the rounded portion 13a of the expanded diameter part 13 is brought into close contact with the inner surface of the connection duct 301, the food which has passed through the esophagus 200 is prevented from moving into the stomach 300 outside the shape-memory alloy stent 10 due to the weight or the speed at which the food is moving. In other words, all the food moves into the shape-memory alloy stent 10 without leaking between the expanded diameter part 13 and the inner surface of the connection duct 301.

The support stent 30 is closely adhered to the inner surface of the cardiac orifice 302 of the stomach 300 by its own elastic force, preventing the shape-memory alloy stent 10 from becoming displaced from the correct position and from completely moving into the stomach 300.

Meanwhile, because the second medical film 13b of the expanded diameter part 13 and the third medical film 34 of the support stent 30 are made of silicone that are plastic and soft and have an elasticity higher than that of PTFE (polyetrafluoroethylene), the support force of the expanded diameter part 13 and the support stent 30 can be further enhanced.

Food that has moved into the cylindrical stent body 15 via the expanded diameter part 13 directly moves from the shape-memory alloy stent 10 into the duodenum 400.

Food that moves into the duodenum 400 along the shape-memory alloy stent 10 mixes with bile coming out of the gall bladder and pancreatic juice coming out of the pancreas and is digested so that nutrients of the food are absorbed by the body.

Thus, when the patient who has undergone a gastrectomy takes in food, the food moves directly into the duodenum 400 via the stent 100 without meeting the stomach 300, thus preventing inflammation of the excised region of the stomach 300, so that a suture region 310 can be safely protected.

Furthermore, food which has moved into the duodenum 400 via the shape-memory alloy stent 10 is digested so that nutrients of the food are absorbed by the body, thus preventing the nutrient supply from becoming imbalanced.

After the suture region 310 has completely healed after a predetermined period of time has passed, the shape-memory alloy stent 10 is removed from the body. For this, the removal string ring 13c connected to the expanded diameter part 13 is pulled by the catheter. Then, the shape-memory alloy stent 10 is reduced in diameter while increasing in length and is inserted into the catheter before being completely removed from the body of the patient.

As described above, a stent of the present invention prevents the stomach of a patient who has undergone a gastrectomy from being brought into direct contact with food, so that the suture region is prevented from being contaminated, thus preventing inflammation of the suture region, and preventing the suture region from being reopened by the inflammation or damage done to the ligation region after the surgery.

Furthermore, the stent prevents food from coming into contact with the suture region so as to avoid the occurrence of inflammation, thus reducing the treatment period.

In addition, the stent includes a medical film which is made of PTFE or silicone, thus preventing food from leaking out of the stent, and enhancing the support performance of the stent. Moreover, the stent may be implanted before the gastrectomy is conducted. In this case, food that has passed through the stomach is also prevented from mixing with bile or pancreatic juice in the duodenum and directly moves into the small intestine to prevent the duodenum from absorbing nutrients of the food while the small intestine directly digests the food and absorbs the nutrients, thus minimizing a nutrient absorption rate, thereby preventing the obesity of the patient.

## Claims

1. A stent, comprising:
a hollow cylindrical stent body (15) formed by weaving at least one first wire (11) made of a super-elastic shape memory alloy such that a plurality of diamond-shaped openings (12) are formed in the cylindrical stent body (15), and an expanded diameter part (13) which is formed on an end of the cylindrical stent body, with bent portions (14) formed along circumferences of both ends of the cylindrical stent body (15), wherein the cylindrical stent body (15) is coated with a first medical film (15a);
**characterized by** further comprising:
a support stent (30) having an elastic spherical structure and formed by weaving at least one second wire (31) or by threading a plurality of second wires (31) to each other in a zigzag manner such that a hollow hole (32) is formed through a central portion of the elastic spherical structure, wherein the or each second wire is made of a super-elastic shape memory alloy and wherein the support stent is fitted, through the hollow hole (32), over a circumferential outer surface of the cylindrical stent body (15) behind the expanded diameter part (13), with coupling wires (33) connecting both ends of the support stent (30) to the cylindrical stent body (15),
wherein the expanded diameter part (13) has a rounded portion (13a) on a junction between the expanded diameter part (13) and the cylindrical stent body (15) and is coated with a second medical film (13b), with a removal string ring (13c) connected to at least one of the bent portions (14) of the expanded diameter part (13),
wherein the support stent (30) is coated with a third medical film (34), and
wherein in use the rounded portion (13a) of the expanded diameter part (13) is caught and supported by an inner surface of a connection tube (301) connected between an esophagus (200) and a stomach (300) of a patient, the support stent (30) is supported by a curved inner surface of a cardiac orifice (302) of the stomach (300), and a second end of the cylindrical stent body (15) is inserted into a pylorus (303) of the patient via a cavity of the stomach (300).

2. The stent as set forth in claim 1, wherein the first medical film (15a) of the hollow cylindrical stent body (15) is made of PTFE (polyetrafluoroethylene) and is formed in a circumferential inner surface or a circumferential outer surface of the first wire (11).

3. The stent as set forth in claim 1, wherein a mesh of the support stent (30) is smaller than a mesh of the hollow cylindrical stent body (15), and the support stent (30) comprises a plurality of support stents (30).

4. The stent as set forth in claim 1, wherein the second and third medical films (13b, 34) are respectively formed by immersing the expanded diameter part (13) and the support stent (30) in liquefied silicone and hardening the silicone applied thereto so that the second and third medical films (13b, 34) cover surfaces of the first wire (11) of the expanded diameter part (13) and the second wire (31) and close the diamond-shaped openings (12) defined by the first wire (11) of the expanded diameter part (13) and the second wire (31) of the support stent (30).

## Patentansprüche

1. Stent, umfassend:
einen hohlen zylindrischen Stentkörper (15), der dadurch gebildet worden ist, dass mindestens ein erster aus einer superelastischen Formgedächtnislegierung hergestellter Draht (11) so gewebt worden ist, dass eine Vielzahl von rautenförmigen Öffnungen (12) im zylindrischen Stentkörper (15) gebildet werden, und einen Teil (13) mit expandiertem Durchmesser, der an einem Ende des zylindrischen Stentkörpers ausgebildet ist, wobei gebogene Abschnitte (14) entlang Umfängen beider Enden des zylindrischen Stentkörpers (15) ausgebildet sind, wobei der zylindrische Stentkörper (15) mit einer ersten medizinischen Folie (15a) beschichtet ist,
**dadurch gekennzeichnet, dass** er ferner Folgendes umfasst:
einen Stützstent (30), der eine elastische kugelförmige Struktur hat und durch Weben mindestens eines zweiten Drahts (31) oder durch zickzackartiges Aneinanderfädeln einer Vielzahl von zweiten Drähten (31), so dass ein hohles Loch (32) durch einen mittleren Abschnitt der elastischen kugelförmigen Struktur gebildet wird, wobei der oder jeder zweite Draht aus einer superelastischen Formgedächtnislegierung hergestellt ist und wobei der Stützstent durch das hohle Loch (32) über einer äußeren Umfangsfläche des zylindrischen Stentkörpers (15) hinter dem Teil (13) mit expandiertem Durchmesser angebracht ist, wobei Koppeldrähte (33) beide Enden des Stützstents (30) mit dem zylindrischen Stentkörper (15) verbinden,
wobei der Teil (13) mit expandiertem Durchmesser an einem Übergang zwischen dem Teil (13) mit expandiertem Durchmesser und dem zylindrischen Stentkörper (15) einen abgerundeten Abschnitt (13a) hat und mit einer zweiten medizinischen Folie (13b) beschichtet ist, wobei ein Entfernungsfadenring (13c) mit mindestens einem der gebogenen Abschnitte (14) des Teils (13) mit expandiertem Durchmesser verbunden ist,
wobei der Stützstent (30) mit einer dritten medizinischen Folie (34) beschichtet ist und
wobei der abgerundete Abschnitt (13a) des Teils (13) mit expandiertem Durchmesser im Gebrauch durch eine innere Fläche einer Verbindungsröhre (301) erfasst und gestützt wird, die zwischen einer Speiseröhre (200) und einem Magen (300) eines Patienten verbunden ist, wobei der Stützstent (30) durch eine gekrümmte innere Fläche eines Magenmunds (302) des Magens (300) gestützt ist und ein zweites Ende des zylindrischen Stentkörpers (15) über einen Hohlraum des Magens (300) in einen Pylorus (303) des Patienten eingeführt wird.

2. Stent nach Anspruch 1, wobei die erste medizinische Folie (15a) des hohlen zylindrischen Stentkörpers (15) aus PTFE (Polytetrafluorethylen) hergestellt ist und in einer inneren Umfangsfläche oder einer äußeren Umfangsfläche des ersten Drahts (11) ausgebildet ist.

3. Stent nach Anspruch 1, wobei ein Netz des Stützstents (30) kleiner als ein Netz des hohlen zylindrischen Stentkörpers (15) ist und der Stützstent (30) eine Vielzahl von Stützstents (30) umfasst.

4. Stent nach Anspruch 1, wobei die zweite und die dritte medizinische Folie (13b, 34) jeweils durch Eintauchen des Teils (13) mit expandiertem Durchmesser und des Stützstents (30) in verflüssigtes Silikon und Härten des darauf aufgebrachten Silikons gebildet sind, so dass die zweite und die dritte medizinische Folie (13b, 34) Flächen des ersten Drahts (11) des Teils (13) mit expandiertem Durchmesser und des zweiten Drahts (31) abdecken und die durch den ersten Draht (11) des Teils (13) mit expandiertem Durchmesser und den zweiten Draht (31) des Stützstents (30) definierten rautenförmigen Öffnungen (12) verschließen.

## Revendications

1. Stent comprenant :
un corps de stent (15) cylindrique creux, formé en tissant au moins un premier fil (11) composé d'un alliage à mémoire de forme superélastique de telle sorte qu'une pluralité d'ouvertures (12) en forme de losanges soient formées dans le corps de stent (15) cylindrique, et une partie à diamètre élargi (13) qui est formée sur une extrémité du corps de stent cylindrique, des portions courbes (14) étant formées le long de circonférences des deux extrémités du corps de stent (15) cylindrique, le corps de stent (15) cylindrique étant revêtu d'un premier film médical (15a) ;
**caractérisé en ce qu'**il comprend en outre :
un stent de support (30) comportant une structure sphérique élastique et formé en tissant au moins un second fil (31) ou en passant une pluralité de seconds fils (31) les uns dans les autres en zigzag de telle sorte qu'un orifice creux (32) soit formé à travers une portion centrale de la structure sphérique élastique, le ou chaque second fil étant composé d'un alliage à mémoire de forme superélastique et le stent de support étant posé, par le biais de l'orifice creux (32), sur une surface circonférentielle extérieure du corps de stent (15) cylindrique derrière la partie à diamètre élargi (13), des fils d'accouplement (33) reliant les deux extrémités du stent de support (30) au corps de stent (15) cylindrique,
la partie à diamètre élargi (13) comportant une portion arrondie (13a) sur une jonction entre la partie à diamètre élargi (13) et le corps de stent (15) cylindrique et étant revêtue d'un deuxième film médical (13b), une boucle de retrait (13c) étant reliée à au moins une des portions courbes (14) de la partie à diamètre élargi (13),
le stent de support (30) étant revêtu avec un troisième film médical (34), et
la portion arrondie (13a) de la partie à diamètre élargi (13) étant, lors de l'utilisation, calée et supportée par une surface intérieure d'un tube de raccordement (301) relié entre un oesophage (200) et un estomac (300) d'un patient, le stent de support (30) étant supporté par une surface intérieure courbe d'un cardia (302) de l'estomac (300), et une seconde extrémité du corps de stent (15) cylindrique étant insérée dans un pylore (303) du patient en passant par une cavité de l'estomac (300).

2. Stent selon la revendication 1, dans lequel le premier film médical (15a) du corps de stent (15) cylindrique creux est composé de PTFE (polytétrafluoroéthylène) et est formé dans une surface circonférentielle intérieure ou une surface circonférentielle extérieure du premier fil (11).

3. Stent selon la revendication 1, dans lequel une maille du stent de support (30) est plus petite qu'une maille du corps de stent (15) cylindrique creux, et le stent de support (30) comprend une pluralité de stents de support (30).

4. Stent selon la revendication 1, dans lequel les deuxième et troisième films médicaux (13b, 34) sont respectivement formés en immergeant la partie à diamètre élargi (13) et le stent de support (30) dans de la silicone liquifiée et en durcissant la silicone appliquée à ceux-ci de telle sorte que les deuxième et troisième films médicaux (13b, 34) recouvrent des surfaces du premier fil (11) de la partie à diamètre élargi (13) et du second fil (31) et ferment les ouvertures en forme de losanges (12) définies par le premier fil (11) de la partie à diamètre élargi (13) et le second fil (31) du stent de support (30).
